Europäisches Patentamt

European Patent Office

Office européen des brevets

(11) Publication number: **0 254 470**
**A1**

# (12) EUROPEAN PATENT APPLICATION.

(21) Application number: **87306232.7**

(22) Date of filing: **14.07.87**

(51) Int. Cl.⁴: **C01F 7/02 , C04B 35/10**

(30) Priority: **18.07.86 GB 8617656**

(43) Date of publication of application:
**27.01.88 Bulletin  88/04**

(84) Designated Contracting States:
**AT BE CH DE FR GB IT LI NL SE**

(71) Applicant: **ALCAN INTERNATIONAL LIMITED**
**1188 Sherbrooke Street West**
**Montreal Quebec H3A 3G2(CA)**

(72) Inventor: **Southern, Jennifer Carolyn**
**61 Middle Road**
**Higher Denham, UB9 5EQ(GB)**

(74) Representative: **Fisher, Bernard et al**
**Raworth, Moss & Cook 36 Sydenham Road**
**Croydon Surrey CR0 2EF(GB)**

(54) **Alumina and ceramics made therefrom.**

(57) Granular alpha-alumina obtained from pseudo-bohmite and haivng a content of non-volatile impurities not exceeding 0.5% and a surface of at least 3m²/g, is disclosed. The grains can produce a fired alumina article of density at least 3.8 g/cm³ on firing for 2 hours at 1500°C after pressing the particles at at least 648 bars. The product may be obtained by calcining pseudo-bohmite of surface area at least 200m²/g.

EP 0 254 470 A1

## ALUMINA AND CERAMICS MADE THEREFROM

This invention relates to a method of making alumina which is highly suitable for use in the manufacture of high-quality alumina ceramics.

It is known that anhydrous alumina particles may be sintered together to produce alumina ceramic articles. Alumina has a higher thermal conductivity than many other metal oxides which are used in ceramics and accordingly alumina ceramics are suitable for applications where thermal shock resistance is required, such as cutting tools. It also has a high electric resistance, making alumina ceramics suitable for use as supports for electric circuits, especially as the high thermal conductivity allows heat generated in the circuits to be dissipated, the ceramic support then acting as a heat sink.

Alumina is also non-toxic and alumina ceramics may be used for various medical prosthetic uses, for example in artificial tooth roots and artificial joints such as hip joints.

High quality alumina ceramic articles should have high strength, low porosity (that is, a high fired density) and a uniform grain structure throughout the article. For many applications it is desirable to obtain a rugosity of a few microns or less, and to achieve such smoothness, the grain structure of the ceramic should be as fine as possible. A small grain size in the ceramic is also desirable in order to achieve high strength and fracture toughness. Firing ceramic articles at high temperatures generally leads to grain growth; thus it is desirable to provide an alumina for ceramic use which is in the form of fine grains and which requires only a low firing temperature to form a ceramic article.

It is also desirable to use an alumina having a high degree of chemical purity as the presence of impurities, including other oxides such as sodium oxide, in the alumina may give unwanted secondary crystal phases, glassy phases or residual porosity due to evolution of gases on firing. All these features have a bad effect on the properties of the fired product.

Alumina used for making ceramics should consist of alpha-alumina (the most stable phase of aluminium oxide at high temperatures). Other alumina phases present, although they may have a very large surface area, do not contribute significantly to the binding together of alumina grains which occurs on firing. It has also been found that the presence of aggregates of non-alpha alumina causes reagglomeration, and therefore incomplete grain size reduction on milling; the non-alpha alumina aggregates may absorb large amounts of binder, which can affect viscosity and drying rates; and it is believed that the presence of non-alpha phases in the material before firing produces high porosity and high local shrinkage. In order to obtain high-quality ceramics comprising 100% alumina it is desirable to use pure alpha-alumina in the form of very small, essentially regular spherical cystals as the material which is fired.

Alpha-alumina is most commonly made by calcining gibbsite, which is an alumina trihydrate, obtained by the Bayer process for extraction of alumina from bauxite. Alumina trihydrate obtained from the Bayer process is contaminated with sodium hydroxide obtained from the strongly alkaline solution used for dissolving the bauxite and from which the alumina trihydrate is then precipitated. The sodium hydroxide can be removed from the alumina trihydrate by washing to only a limited extent, and elaborate further processing is required to obtain alumina having the degree of purity desired for making alumina ceramics. Moreover, the manner of precipitation of the alumina trihydrate from the alkaline solution has to be controlled if complete conversion of the alumina trihydrate to alpha alumina is to be achieved on calcining at a temperature low enough to avoid undesirable grain growth, otherwise intermediate non-alpha transition phases are present in the calcined product.

High purity alumina may be prepared by other methods, such as calcining ammonium alum or aluminium nitrate, but it has been found that alpha-alumina obtained by these methods has poor sintering properties when fired to give a ceramic body. Alpha-alumina may also be made by calcining crystalline bohmite (bohmite in the form of relatively large crystalline obtained by hydrothermal digestion of alumina, but the resulting alpha-alumina again gives an unsatisfactory ceramic product on firing.

Japanese Patent Application No. 58.135126 discloses ultramicro Al(OH)$_3$ obtained by hydrolysis of an aluminium alkoxide containing residual carbon which, after mixing with magnesium oxide and calcining, gives an alumina ceramic of high density after firing in vacuo at 1850°C.

Japanese Patent Application No. 81.144413 discloses that alumina ceramics in the form of tubes or long rods may be obtained by compressing alumina of extremely high purity (99.99%) and small grain size and mixed with a sintering aid, a moulding aid and an additive to shape, heating at 1000°C to remove the moulding agents and firing under vacuum at 1900°C.

British Patent Application No. 2071073 describes a process in which alumina of small grain size containing magnesium and zirconium and/or hafnium oxides is formed to shape and fired in hydrogen at a high temperature (typically 1880°C).

2

French Patent Application No. 2235086 describes milling of alumina in the presence of iron and/or chromium oxides followed by calcination, further milling, pressing to shape and firing. When bohmite is used as a starting material its surface area does not exceed 152 m²/g and the content of sodium oxide does not exceed 0.01%.

Japanese Patent Application No. 80.113615 describes a process in which raw alumina is pulverized, calcined until 75% of the alumina is converted to alpha-alumina, ground again, formed to shape and fired.

British Patent Application No. 2108949 describes a process in which aluminium hydroxide is activated by heating to 500-600°C, washed to remove excess alkali, and calcined in the presence of a mineralizing mixture such as boric acid/aluminium fluoride/calcium fluoride.

It has now been discovered that alpha-alumina capable of producing excellent pure alumina ceramics at a relatively low firing temperature may be obtained by calcining a micro-crystalline pseudo-bohmite comprising very fine crystals.

According to one aspect of the invention, there is provided a method of making alpha-alumina which comprises calcining a pseudo-bohmite having a surface area of at least 200 m²/g and a content of non-volatile impurities (as hereinafter defined) not exceeding 0.5% by weight on the $Al_2O_3$ content to convert substantially all the pseudo-bohmite to alpha-alumina grains having a surface area of at least 3 m²/g.

The "non-volatile impurities" mentioned above are $SiO_2$, $Na_2O$, $Fe_2O_3$, $CaO$ and $TiO_2$. The content of these impurities is measured by weight on the total amount of dry $Al_2O_3$ excluding any bound and unbound water, present in the pseudo-bohmite. It has been found that the presence (in reasonably small amounts) of volatile impurities such as sulphate, chloride and nitrate does not affect the desired properties of the product obtained as these are removed by evaporation on calcining and/or subsequent sintering. A grain growth inhibitor such as MgO may be present in the pseudo-bohmite and is not considered as an impurity.

The surface area of the pseudo-bohmite is preferably at least 230 m²/g, most preferably at least 250 m²/g and the content of non-volatile impurities is preferably no more than 0.4%, most preferably no more than 0.3% by weight. The content of $Na_2O$ as an impurity is preferably not more than 0.4%, most preferably not more than 0.1%.

The surface area of the alpha-alumina produced is preferably from 3 to 35 m²/g and most preferably from 3 to 15 m²/g.

According to another aspect of the invention, a method of making an alumina ceramic body comprises forming an article of grains of such an alumina to shape and firing the article at a maximum temperature from 1450°C to 1750°C.

The invention also relates to alpha-alumina grains obtained from pseudo-bohmite having a content of non-volatile impurities (as defined above) not exceeding 0.5% by weight of the dry $Al_2O_3$ content and a surface area of at least 3 m²/g, the grains being capable of producing a fired alumina article having a density of at least 3.8 g/cm³, preferably at least 3.9 g/cm³ on firing for 2 hours at 1500°C after pressing the particles together at a pressure of 4 tsi (618 bars).

The surface area is that measured by the standard Strohlein method as described in "Particle Size Measurement", P.390, Terence Allen, Chapman & Hall Limited, 1975. It has been found that the alpha-alumina produced according to the invention, when formed to shape and fired to give a ceramic article, is capable of giving a ceramic article having excellent strength and uniformity, a very high degree of surface smoothness and a density which is close to the theoretical maximum density of 3.98g/cc. The firing temperature and firing duration required to obtain the sintering necessary to produce the ceramic are sufficiently low to avoid undesirable grain growth which would cause deterioration in the surface state of the article produced.

When calcining the pseudobohmite the maximum temperature reached is preferably 1125°C to 1425°C, most preferably 1225°C to 1375°C. It has been found that a maximum calcination temperature from 1125°C to 1425°C is sufficient to achieve conversion of the pseudobohmite to an alpha alumina having a high degree of reactivity which is required for making a ceramic having the desired properties. The soak time (the time at which the psuedobohmite is held at maximum temperature during calcination) required depends on the calcination temperature, being lower at higher temperatures, and the preferred soak time is 0-30 hours, most preferably 1-15 hours. A soak time of one hour has been found adequate at soak temperatures of above 1225°C. Better results are obtained when the calcinationis carried out at relatively low heating and cooling rates. Calcination may be carried out using conventional methods and equipment.

A proportion of the alpha-alumina grains obtained on calcining the pseudo-bohmite are generally present as agglomerates and it is normally desirable to break up these agglomerates before the alpha-alumina is formed into an article for firing. The agglomerates may be broken up by passing the alpha-alumina through an attrition mill, such as a ball mill loaded with alumina balls.

3

It has been found that the alpha-alumina made by the above-mentioned method is capable of giving fired articles having a density after firing of at least 3.8g/cm³, and often at least 3.9 g/cm³ on firing for 2 hours at 1500°C after pressing the alpha-alumina particles at a pressure of 4 tons per square inch (618 bars). In order to make a fired article the alpha-alumina may be mixed with a fugitive binder, such as an aqueous solution of polyethylene glycol, sieved if necessary to break up agglomerates, and pressed in a conventional press to produce a green body of the required shape, which is then dried and fired.

It has been found that the required purity of the pseudo-bohmite to produce a given density on firing of the alpha-alumina depends on the surface area of the pseudo-bohmite, and decreases as the surface area increases. If the surface area is 200 m²/g a content of non-volatile impurities not exceeding 0.1% is desirable but at a surface area of 270 m²/g an impurity level of 0.5% is acceptable. In order to obtain a fired density of 3.9 g/cm³ at a surface area of 230 m²/g an impurity level not exceeding 0.1% is generally required, but at an area of 300 m²/g an impurity level of 0.3% still gives good results.

The density of the fired body obtained depends on the firing cycle used and also on the pressure used for forming the green body to shape. The density obtained generally increases with increased forming pressure, increased maximum temperature on firing and increased duration of firing; however an excessively high firing temperature gives excessive undesired grain growth. The maximum temperature reached on firing may be from 1450°C to 1750°C and, depending on the firing temperature, the soak time at maximum temperature may be from 0 to 16 hours. In order to achieve a satisfactory fired density a soak time of 8 hours at 1450°C may be used, at 1500°C a soak time of 2 hours is generally adequate, and lower soak times may be used at higher temperatures. The pressure used for forming may be about 2 tsi (309 bars) to 4 tsi (618 bars). Firing may be carried out in air by conventional methods.

The pseudo-bohmite starting material may be obtained by various methods, including precipitation from an aqueous solution of alum and decomposition of an aluminium alkoxide. Pseudo-bohmites of suitable surface area and purity are available commercially.

Method of making alpha-alumina and the products obtained will now be described by way of illustration in the following Examples.

## EXAMPLES

The pseudo-bohmites used are those given in Table 1 which gives their surface areas (measured by the Strohlein method) and their contents of impurities as weight % on the dry Al₂O₃ content. Pseudo-bohmites Nos. 4, 5, 7 and 8 are comparison examples.

Samples of the pseudo-bohmites 1 and 2 are calcined in an electric furnace while contained in lidded recrystallized alumina crucibles, under the conditions given in Table 2 in which the "soak time" is time at maximum temperature and "heating and cooling" gives the average rate of heating and cooling. After calcination the alumina produced is milled in a ball mill containing debased alumina balls of diameter from 1.3 to 2.5 cm at a ball to charge ratio of 40:1 with 0.5% by weight on the alumina charge of napthenic acid added as a milling aid. The surface areas of the products obtained, after calcination and after milling, are shown in Table 2.

The alpha aluminas produced are then mixed with an aqueous solution of polyethylene glycol as a fugitive binder, sieved to break up agglomerates, pressed uniaxially into pellets at a pressure of 2 tsi (309 bars) or 4 tsi (618 bars, and dried at 110°C for at least 2 hours. The green densities obtained are shown in Table 2. The green pellets so produced are then fired in air at a heating and cooling rate of 5°C/minute and a soak time at maximum temperature of 2 hours. The maximum temperatures and the fired densities obtained are shown in Table 2. In addition, pressed samples of alpha alumina from pseudobohmite 2 are fired under the conditions given in Table 3 which gives the fired densities obtained.

Samples of all the alpha aluminas obtained from the pseudobohmites of Table 1 are likewise pressed and fired, using the same conditions as for Table 2, and the results obtained are shown in Table 4. Table 4 also shows the results obtained with a pseudobohmite starting material having a surface area of 1 m²/g calcined at 1200°C (line 15).

Table 5 shows the results obtained in like manner with some of the pseudobohmites mentioned in Table 1 with deliberate additions of other oxides representing common impurities found in alumina.

The results given in these Tables show that pseudobohmites having an initial surface area of less than 200 m²/g and/or a content of non-volatile impurities greater than 0.5% give poor fired densities. Superior results are obtained with surface areas in excess of 230 m²/g, most preferably in excess of 250 m²/g, and non-volatile impurity contents of less than 0.3%. Better results are obtained at low heating and cooling rates during calcination. The firing temperature required to achieve a satisfactory density is relatively low.

The fired articles obtained have a high strength, small grain size, a uniform grain structure and a low rugosity. They are highly suitable for use as support in electric circuits and for other uses.

**TABLE 1**

| Pseudobohmite | Surface Area $m^2g^{-1}$ | Purity wt% on $Al_2O_3$ | | | | | | 1500°C 4 tsi Fired Density $gcm^{-3}$ |
|---|---|---|---|---|---|---|---|---|
| | | $Na_2O$ | $SiO_2$ | $Fe_2O_3$ | $CaO$ | $TiO_2$ | Sum | |
| 1 | 307 | 0.04 | 0.061 | 0.043 | 0.061 | | 0.205 | 3.92 |
| 2 | 279 | 0.017 | 0.084 | 0.059 | 0.048 | 0.008 | 0.216 | 3.92 |
| 3 | 226 | 0.007 | 0.149 | 0.037 | 0.007 | | 0.2 | 3.80 |
| 4 | 223 | 0.58 | 0.11 | 0.022 | 0.27 | | 0.982 | 3.53 |
| 5 | 223 | 0.58 | 0.067 | 0.020 | 0.08 | 0.006 | 0.753 | 3.71 |
| 6 | 223 | 0.02 | 0.06 | 0.014 | 0.05 | 0.002 | 0.146 | 3.84 |
| 7 | 100 | 0.025 | 0.15 | 0.027 | 0.005 | | 0.207 | 3.62 |
| 8 | 312 | 0.007 | 0.406 | 0.027 | 0.20 | 0.006 | 0.646 | 3.87 |

TABLE 1 (Cont'd)

| Pseudobohmite | Surface Area $m^2g^{-1}$ | Purity wt% on $Al_2O_3$ | | | | | | $1500^{\circ}C$ 4 tsi Fired Density $gcm^{-3}$ |
|---|---|---|---|---|---|---|---|---|
| | | $Na_2O$ | $SiO_2$ | $Fe_2O_3$ | CaO | $TiO_2$ | Sum | |
| 9 | 285 | 0.012 | 0.059 | 0.054 | 0.024 | 0.003 | 0.152 | 3.92 |
| 10 | 141 | 0.007 | 0.032 | 0.021 | 0.040 | 0.012 | 0.112 | 3.29 |
| 11 | 240 | 0.041 | 0.120 | 0.017 | 0.031 | 0.002 | 0.211 | 3.83 |
| 12 | 259 | 0.32 | 0.28 | 0.019 | 0.040 | 0.003 | 0.662 | 3.86 |
| 13 | 266 | 0.075 | 0.32 | 0.021 | 0.030 | 0.004 | 0.450 | 3.70 |
| 14 | 261 | 0.013 | 0.43 | 0.024 | 0.260 | 0.003 | 0.730 | 3.86 |

## TABLE 2

| Pseudo-bohmite | Calcination Temperature | Soak Time | Heating and Cooling | Surface Area | Milled Surface Area | Pressure | Green Density | Fired Density gcm⁻³ | | |
|---|---|---|---|---|---|---|---|---|---|---|
| | | | | | | | | 1500°C | 1550°C | 1650°C |
| | °C | hr | °C min$^{-1}$ | m²g$^{-1}$ | m²g$^{-1}$ | tsi | gcm$^{-3}$ | | | |
| 1 | 1175 | 1 | 1 | 14.0 | 13.0 | 2<br>4 | 1.93<br>2.00 | 3.89<br>3.90 | 3.91<br>3.92 | 3.91<br>3.92 |
| | 1225 | 1 | 1 | 5.9 | 8.2 | 2<br>4 | 2.05<br>2.14 | 3.85<br>3.90 | 3.92<br>3.95 | 3.95<br>3.95 |
| | 1275 | 1 | 1 | 5.7 | 6.3 | 2<br>4 | 2.08<br>2.14 | 3.90<br>3.92 | 3.94<br>3.95 | 3.94<br>3.94 |
| | 1325 | 1 | 1 | 5.3 | 7.8 | 2<br>4 | 2.13<br>2.18 | 3.91<br>3.92 | 3.92<br>3.94 | 3.92<br>3.93 |
| 2 | 1275 | 0 | 1 | 7.6 | 8.3 | 2<br>4 | 1.99<br>2.05 | 3.86<br>3.90 | 3.91<br>3.93 | 3.91<br>3.93 |
| | 1275 | 1 | 1 | 6.1 | 7.0 | 2<br>4 | 2.04<br>2.10 | 3.91<br>3.92 | 3.93<br>3.95 | 3.93<br>3.94 |
| | 1275 | 5 | 1 | 6.9 | 7.2 | 2<br>4 | 2.05<br>2.11 | 3.89<br>3.92 | 3.93<br>3.94 | 3.94<br>3.95 |
| | 1275 | 10 | 1 | 6.0 | 7.3 | 2<br>4 | 2.05<br>2.11 | 3.89<br>3.92 | 3.92<br>3.94 | 3.92<br>3.93 |
| | 1275 | 15 | 1 | 6.1 | 7.1 | 2<br>4 | 2.11<br>2.17 | 3.92<br>3.94 | 3.94<br>3.95 | 3.94<br>3.95 |
| | 1275 | 30 | 1 | 6.6 | 6.4 | 2<br>4 | 2.11<br>2.17 | 3.92<br>3.94 | 3.94<br>3.95 | |
| | 1125 | 1 | 1 | 40.0 | 33.0 | 2<br>4 | 1.76<br>1.86 | 3.78<br>3.85 | 3.83<br>3.89 | 3.89<br>3.92 |

TABLE 2 - Continued

| 2 | 1325 | 1 | 1 | 7.3 | 8.2 | 2 | 2.04 | 3.90 | | |
|---|------|---|-----|------|------|--------|--------------|--------------|--------------|--------------|
| | 1375 | 1 | 1 | 5.2 | 7.0 | 2<br>4 | 2.11<br>2.17 | 3.91<br>3.93 | 3.94<br>3.96 | 3.94<br>3.95 |
| | 1275 | 1 | 0.5 | 7.3 | 8.4 | 2<br>4 | 2.04<br>2.11 | 3.92<br>3.94 | 3.92<br>3.95 | |
| | 1275 | 1 | 1:0 | 7.3 | 8.3 | 2<br>4 | 2.03<br>2.09 | 3.90<br>3.93 | 3.92<br>3.93 | 3.93<br>3.94 |
| | 1275 | 1 | 2.0 | 7.7 | 8.8 | 2<br>4 | 2.00<br>2.06 | 3.88<br>3.91 | 3.91<br>3.93 | 3.93<br>3.94 |
| | 1275 | 1 | 20.0 | 6.3 | 8.4 | 2<br>4 | 1.99<br>2.06 | 3.86<br>3.90 | 3.91<br>3.93 | 3.91<br>3.92 |
| | 1425 | 1 | 1 | 4.0 | 5.2 | 2<br>4 | 2.17<br>2.23 | 3.88<br>3.91 | 3.93<br>3.94 | |
| | 1475 | 1 | 1 | 2.9 | 4.2 | 2<br>4 | 2.20<br>2.27 | 3.70<br>3.77 | 3.86<br>3.89 | |
| | 1125 | 5 | 1 | 14.7 | 13.0 | 2<br>4 | 1.93<br>2.01 | 3.85<br>3.90 | 3.88<br>3.91 | |

0 254 470

8

TABLE 2 (Cont'd)

| Pseudoboehmite | Calcination Tempearture °C | Soak Time hr | Heating and Cooling | Surface Area $m^2g^{-1}$ | Milled Surface $m^2g^{-1}$ | Pressure tsi | Green Density $gcm^{-3}$ | Fired Density $gcm^{-3}$ | | |
|---|---|---|---|---|---|---|---|---|---|---|
| | | | | | | | | 1500°C | 1550°C | 1650°C |
| 2 | 1125 | 4 | 1 | 24.1 | 15.2 | 2 | 1.85 | 3.85 | 3.87 | 3.90 |
| | | | | | | 4 | 1.93 | 3.89 | 3.91 | 3.92 |
| | 1125 | 3 | 1 | 16.8 | 14.7 | 2 | 1.91 | 3.86 | 3.88 | 3.89 |
| | | | | | | 4 | 1.98 | 3.89 | 3.91 | 3.92 |
| | 1125 | 2 | 1 | 12.5 | 14.8 | 2 | 1.86 | 3.85 | 3.89 | 3.90 |
| | | | | | | 4 | 1.95 | 3.89 | 3.91 | 3.92 |
| | 1125 | 1 | 1 | 21.2 | 22.1 | 2 | 1.84 | 3.84 | 3.87 | 3.89 |
| | | | | | | 4 | 1.93 | 3.91 | 3.91 | 3.92 |
| | 1225 | 12 | 1 | 6.5 | 7.3 | 2 | 2.04 | 3.90 | 3.92 | 3.92 |
| | | | | | | 4 | 2.10 | 3.93 | 3.94 | 3.93 |
| | 1325 | 12 | 1 | 5.0 | 6.2 | 2 | 2.13 | 3.91 | 3.94 | 3.93 |
| | | | | | | 4 | 2.19 | 3.93 | 3.95 | 3.93 |
| | 1375 | 12 | 1 | 3.8 | 4.8 | 2 | 2.18 | 3.85 | 3.92 | 3.94 |
| | | | | | | 4 | 2.23 | 3.88 | 3.94 | 3.94 |
| 9 | 1325 | 10 | 1 | 4.4 | 5.8 | 2 | 2.11 | 3.90 | 3.94 | 3.92 |
| | | | | | | 4 | 2.16 | 3.92 | 3.95 | 3.92 |

TABLE 3

| Alumina | Surface Area | Milled Surface Area | Pressure | Green Density | Firing Temperature | Soak Time | Heating and Cooling Rate | Fired Density |
|---|---|---|---|---|---|---|---|---|
| | $m^2g^{-1}$ | $m^2g^{-1}$ | tsi | $gcr^{-3}$ | °C | hrs | °C $min^{-1}$ | $gcm^{-3}$ |
| 2 1275°C 1 hr | 6.1 | 7.0 | 2 | 2.04 | 1450 | 2 | 5 | 3.79 |
| | | | | | 1450 | 8 | 5 | 3.90 |
| | | | | | 1500 | 2 | 5 | 3.91 |
| | | | | | 1550 | 2 | 5 | 3.93 |
| | | | | | 1600 | 2 | 5 | 3.93 |
| | | | | | 1650 | 2 | 5 | 3.93 |
| | | | | | 1700 | 2 | 5 | 3.93 |
| | | | 4 | 2.10 | 1450 | 2 | 5 | 3.84 |
| | | | | | 1500 | 2 | 5 | 3.92 |
| | | | | | 1550 | 2 | 5 | 3.95 |
| | | | | | 1600 | 2 | 5 | 3.94 |
| | | | | | 1650 | 2 | 5 | 3.94 |
| | | | | | 1700 | 2 | 5 | 3.94 |
| 2 1325°C 1 hr | 7.3 | 8.2 | 2 | 2.04 | 1400 | 16 | 5 | 3.82 |
| | | | | | 1450 | 8 | 5 | 3.89 |
| | | | | | 1450 | 16 | 5 | 3.91 |
| | | | | | 1500 | 2 | 5 | 3.90 |
| | | | | | 1500 | 2 | 0.5 | 3.90 |
| | | | | | 1500 | 2 | 10 | 3.91 |
| | | | | | 1500 | 2 | 20 | 3.91 |
| | | | | | 1500 | 0 | 5 | 3.82 |
| | | | | | 1500 | 4 | 5 | 3.92 |
| | | | | | 1500 | 8 | 5 | 3.93 |
| | | | | | 1500 | 1 | 5 | 3.89 |

TABLE 4

| Pseudobohmite | Surface Area | Milled Surface Area | Forming Pressure | Green Density | Firing Temperature °C | | | |
| --- | --- | --- | --- | --- | --- | --- | --- | --- |
| | | | | | 1500 | 1550 | 1600 | 1650 |
| | $m^2g^{-1}$ | $m^2g^{-1}$ | tsi | $gcm^{-3}$ | Fired Density $gcm^{-3}$ | | | |
| 1 | 5.7 | 6.3 | 2<br>4 | 2.08<br>2.14 | 3.90<br>3.92 | 3.94<br>3.95 | 3.95<br>3.94 | 3.94<br>3.94 |
| 2 | 6.1 | 7.0 | 2<br>4 | 2.04<br>2.10 | 3.91<br>3.92 | 3.93<br>3.95 | 3.90<br>3.91 | 3.93<br>3.93 |
| 3 | 9.4 | 10.5 | 2<br>4 | 1.92<br>1.98 | 3.74<br>3.80 | 3.82<br>3.86 | 3.84<br>3.88 | 3.85<br>3.88 |
| 4 | 6.0 | 7.2 | 2<br>4 | 1.92<br>2.00 | 3.47<br>3.53 | 3.72<br>3.72 | 3.79<br>3.79 | 3.80<br>3.80 |
| 5 | 7.6 | 8.7 | 2<br>4 | 1.89<br>1.97 | 3.67<br>3.71 | 3.75<br>3.78 | 3.80<br>3.82 | 3.83<br>3.84 |
| 6 | 9.6 | 9.9 | 2<br>4 | 1.91<br>1.98 | 3.82<br>3.84 | 3.85<br>3.87 | 3.88<br>3.89 | 3.90<br>3.91 |
| 7 | 7.6 | 7.4 | 2<br>4 | 1.77<br>1.84 | 3.55<br>3.62 | 3.78<br>3.81 | 3.88<br>3.90 | 3.92<br>3.93 |
| 8 | 7.6 | 8.9 | 2<br>4 | 1.87<br>1.96 | 3.85<br>3.87 | 3.87<br>3.89 | 3.87<br>3.88 | 3.87<br>3.87 |
| 9 | 4.4 | 5.8 | 2<br>4 | 2.11<br>2.16 | 3.90<br>3.92 | 3.94<br>3.95 | 3.94<br>3.94 | 3.92<br>3.92 |

0 254 470

## TABLE 4 (Cont'd)

| Pseudoboehmite | Surface Area $m^2g^{-1}$ | Milled Surface Area $m^2g^{-1}$ | Forming Pressure tsi | Green Density $gcm^{-3}$ | Firing Temperature °C | | | |
|---|---|---|---|---|---|---|---|---|
| | | | | | 1500 | 1550 | 1600 | 1650 |
| | | | | | Fired Density $gcm^{-3}$ | | | |
| 10 | 4.1 | 5.3 | 2 | 2.17 | 3.23 | 3.46 | 3.68 | 3.76 |
| | | | 4 | 2.23 | 3.29 | 3.51 | 3.70 | 3.78 |
| 11 | 5.5 | 6.5 | 2 | 2.09 | 3.79 | 3.88 | 3.89 | 3.90 |
| | | | 4 | 2.15 | 3.83 | 3.90 | 3.91 | 3.91 |
| 12 | 8.2 | 10.9 | 2 | 1.88 | 3.82 | 3.84 | 3.85 | 3.86 |
| | | | 4 | 1.96 | 3.86 | 3.86 | 3.87 | 3.88 |
| 13 | 11.3 | 13.1 | 2 | 1.79 | 3.63 | 3.79 | 3.84 | 3.85 |
| | | | 4 | 1.87 | 3.70 | 3.83 | 3.86 | 3.86 |
| 14 | 6.6 | 8.6 | 2 | 1.88 | 3.84 | 3.87 | 3.87 | 3.85 |
| | | | 4 | 1.97 | 3.86 | 3.88 | 3.88 | 3.86 |
| 15* | 2.7 | 5.0 | 4 | 2.11 | 3.15 | 3.61 | 3.78 | 3.82 |

*Pseudoboehmite with surface area 1 $m^2g^{-1}$ calcined at 1200°C.

0.254 470

0 254 470

TABLE 5 - EFFECT OF IMPURITIES

| Pseudoboehmite | Impurity Added | Purity Sum % on $Al_2O_3$ | 4 tsi Green Density | 4 tsi 1500°C Fired Density $gcm^{-3}$ |
|---|---|---|---|---|
| 2 $279\ m^2g^{-1}$ | None | 0.216 | 2.10 | 3.92 |
| | $SiO_2$ | 0.259 | 2.13 | 3.91 |
| | $SiO_2$ | 0.342 | 2.16 | 3.89 |
| | $SiO_2$ | 0.572 | 2.11 | 3.84 |
| | $SiO_2$ | 0.742 | 2.13 | 3.82 |
| | $Na_2O$ | 0.329 | 2.09 | 3.87 |
| | $Na_2O$ | 0.519 | 2.16 | 3.82 |
| | $Na_2O + SiO_2$ | 0.379 | 2.14 | 3.88 |
| | $Na_2O + SiO_2$ | 0.485 | 2.15 | 3.86 |
| | $Na_2O + SiO_2$ | 0.505 | 2.13 | 3.85 |
| | $CaO$ | 0.258 | 2.16 | 3.92 |
| | $CaO$ | 0.408 | 2.20 | 3.89 |
| | $CaO$ | 0.568 | 2.16 | 3.86 |
| | $CaO + SiO_2$ | 0.376 | 2.08 | 3.91 |
| | $CaO + SiO_2$ | 0.434 | 2.06 | 3.90 |
| | $CaO + SiO_2$ | 0.754 | 2.10 | 3.87 |
| 9 $285\ m^2g^{-1}$ | None | 0.152 | 2.16 | 3.92 |
| | $SiO_2$ | 0.294 | 2.10 | 3.89 |
| | $SiO_2$ | 0.373 | 2.08 | 3.86 |
| | $CaO$ | 0.256 | 2.12 | 3.91 |
| | $CaO$ | 0.312 | 2.18 | 3.87 |
| | $CaO + SiO_2$ | 0.328 | 2.04 | 3.90 |
| | $Na_2O$ | 0.227 | 2.14 | 3.88 |
| | $Fe_2O_3$ | 0.238 | 2.16 | 3.91 |
| 10 $141\ m^2g^{-1}$ | None | 0.112 | 2.23 | 3.29 |
| | $Na_2O$ | 0.195 | 2.22 | 3.31 |
| | $Fe_2O_3$ | 0.241 | 2.25 | 3.38 |

**Claims**

1. Granular alpha-alumina obtained from pseudo-bohmite and having a content of non-volatile impurities (as hereinbefore defined) not exceeding 0.5% by weight of the dry $Al_2O_3$ content and a surface area of at least 3 m²/g, the grains being capable of producing a fired alumina article having a density of at least 3.8 g/cm³ on firing for 2 hours at 1500°C after pressing the particles together at a pressure of at least 618 bars.

2. Alpha-alumina according to claim 1, in which the grains are capable of producing a fired alumina article having a density of at least 3.9 g/cm³ on firing for 2 hours at 1500°C after pressing the particles together at a pressure of at least 618 bars.

3. A method of making alpha-alumina which comprises calcining a pseudo-bohmite having a surface area of at least 200 m²/g and a content of non-volatile impurities (as hereinbefore defined) not exceeding 0.5% by weight on the $Al_2O_3$ content to convert all the pseudo-bohmite to alpha-alumina grains having a surface area of at least 3 m²/g.

4. A method according to claim 3, in which the content of sodium oxide does not exceed 0.4% by weight on the $Al_2O_3$ content.

5. A method according to claim 3 or 4, in which the pseudo-bohmite has a surface area of at least 270 m²/g.

6. A method according to claim 3 or 4, in which the pseudo-bohmite has a content of non-volatile impurities not exceeding 0.3% by weight.

7. A method according to claim 3 or 4, in which the pseudo-bohmite has a content of non-volatile impurities not exceeding 0.1% by weight.

8. A method according to claim 3, in which the pseudo-bohmite has a surface area of at least 230 m$^2$/g.

9. A method according to claim 3 or 4, in which the pseudo-bohmite has a surface area of at least 300 m$^2$/g and a content of non-volatile impurities not exceeding 0.3% by weight.

10. A method according to any one of claims 3 to 9, in which the alpha-alumina obtained is milled.

11. A method according to any one of claims 3 to 10, in which the maximum temperature during calcination is from 1125 to 1425°C.

12. A method of making a ceramic article, which comprises forming a shaped body of alpha-alumina according to claim 1 or 2 or made by a method according to any one of claims 3 to 11 and firing the shaped body.

13. A method according to claim 12, in which the body is fired at maximum temperature from 1450 to 1750°C.

## European Patent Office

## EUROPEAN SEARCH REPORT

Application number

EP 87 30 6232

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 100, no. 2, January 1984, page 304, abstract no. 11677K, Columbus, Ohio, US; & JP-A-58 135 126 (MITSUI TOATSU CHEMICALS, INC.) 11-08-1983 * Abstract * | 1-3,8, 9,11, 12 | C 01 F 7/02 C 04 B 35/10 |
| A | CHEMICAL ABSTRACTS, vol. 96, no. 14, April 1982, page 313, abstract no. 109125c, Columbus, Ohio, US; & JP-A-81 144 113 (NGK INSULATORS, LTD) 10-11-1981 * Abstract * | 1,2 | |
| A | GB-A-2 071 073 (GENERAL ELECTRIC) | | |
| A | FR-A-2 235 086 (ROBERT BOSCH) * Pages 4,5 * | 1,10, 11,13 | **TECHNICAL FIELDS SEARCHED (Int. Cl.4)** C 01 F C 04 B |
| A | CHEMICAL ABSTRACTS, vol. 94, no. 18, May 1981, page 301, abstract no. 144316w, Columbus, Ohio, US; & JP-A-80 113 615 (SUMITOMO ALUMINIUM SMELTING CO., LTD) 02-09-1980 | | |

The present search report has been drawn up for all claims

| Place of search | Date of completion of the search | Examiner |
|---|---|---|
| THE HAGUE | 26-10-1987 | LIBBERECHT-VERBEECK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82